(19) Europäisches Patentamt
European Patent Office
Office européen des brevets

(11) Veröffentlichungsnummer: **0 000 348**
**B1**

(12) # EUROPÄISCHE PATENTSCHRIFT

(21) Anmeldenummer: **78100256.3**

(22) Anmeldetag: **28.06.78**

(51) Int. Cl.³: **C 07 C 127/24,**
C 08 G 18/80

(54) **Polyuret-Polyisocyanate, Verfahren zu deren Herstellung, sowie deren Verwendung bei der Herstellung von Polyurethankunststoffen**

(30) Priorität: **06.07.77 DE 2730513**

(43) Veröffentlichungstag der Anmeldung:
**24.01.79 Patentblatt 79/02**

(45) Bekanntmachung des Hinweises auf die Patenterteilung:
**23.07.80 Patentblatt 80/15**

(84) Benannte Vertragsstaaten:
**BE DE FR GB NL SE**

(56) Entgegenhaltungen:
**DE - B - 1 227 004**
**US - A - 3 284 479**

(73) Patentinhaber: **Bayer AG**
**Zentralbereich Patente, Marken und Lizenzen**
**D - 5090 Leverkusen 1, Bayerwerk (DE)**

(72) Erfinder: **Reichmann, Wolfgang, Dr.**
**Vohwinkelallee 19**
**D - 4000 Düsseldorf (DE)**
**König, Klaus, Dr.**
**Heymannstrasse 50**
**D - 5090 Leverkusen (DE)**
**Nordmann, Heinz-Georg, Dr.**
**Erfurter Strasse 6**
**D - 5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 348 B1

# Polyuret-Polyisocyanate, Verfahren zu deren Herstellung, sowie deren Verwendung bei der Herstellung von Polyurethankunststoffen

Die vorliegende Erfindung betrifft neue, Polyuretgruppen aufweisende organische Polyisocyanate, ein neuartiges Verfahren zu deren Herstellung und die Verwendung der neuen Verbindungen als Isocyanat-Komponente bei der Herstellung von Polyurethankunststoffen.

Biuretgruppen enthaltende Polyisocyanate sind bekannt und finden als Rohstoffe für hochwertige, litchtechte Lackierungen praktische Verwendung. Sie können beispielsweise aus Diisocyanaten und Wasser (DT—AS 1 101 394), Schwefelwasserstoff (DT—AS 1 165 580), Ameisensäure (DT—AS 1 174 760), tertiären Alkoholen (DT—AS 1 543 178, DT—AS 1 931 055, Monoaminen (DT—OS 2 308 015) oder Polyaminen (DT—OS 2 261 065) hergestellt werden.

Diese Verfahren des Standes der Technik zur Herstellung von Biuretpolyisocyanaten weisen eine Reihe von Natchteilen auf.

So entstehen bei den meisten der Verfahren aus einem Teil der Isocyanatgruppen zunächst Aminogruppen, die mit überschüssigem Diisocyanat über die entsprechenden Diisocyanatharnstoffe zu Biuretpolyisocyanaten weiterreagiern. Die Umwandlung der Isocyanatgruppen in Aminogruppen wird stets durch die Entstehung von gasförmigen Nebenprodukten wie Kohlendioxid, Kohlenmonoxid, Kohlensulfoxid oder Olefine begleitet, deren Beseitigung zu Abgasproblemen führen kann. Bei der heterogenen Reaktion von Diisocyanaten mit Wasser besteht zusätzlich die Gefahr der Bildung von unlöslichen Polyharnstoffen die nur schwer abzutrennen sind. Die Reaktion von Diisocyanaten mit tertiären Alkoholen, die zur Herstellung von Biuretpolyisocyanaten technisch genutzt wird, hat den weiteren Nachteil, daß für die Urethanspaltung zur Überführung der Isocyanatgruppen in Aminogruppen verhältnismäßig hohe Temperaturen notwendig sind. Von besonderem Nachteil ist jedoch der Umstand, daß bei diesen Verfahren zunächst ein Teil der Isocyanatgruppen des als Ausgangsmaterial verwendeten Diisocyanats unter Aminbildung vernichtet werden muß.

Die direkte Umsetzung von Polyaminen mit Diisocyanaten führt zwar gemäß DT—OS 2 261 065 ohne Abspaltung von flüchtigen Nebenprodukten und ohne Umwandlung von Isocyanatgruppen in Aminogruppen zu Biuretpolyisocyanaten. Bei diesem Verfahren treten aber insbesondere bei Verwendung von technisch leicht zugänglichen Ausgangsmaterialien wie Hexamethylendiamin und Hexamethylendiisocyanat erhebliche praktische Schwierigkeiten auf, da wegen der hohen Reaktivität der Aminogruppen gegenüber den Isocyanatgruppen die Tendenz zur Bildung von unlöslichen Polyharnstoffen sehr groß ist und so in den meisten Fällen zur Vervollständigung der Umsetzung unwirtschaftlich langes Nachheizen des Reaktionsgemisches bei hoher Temperatur notwendig ist, was zu einer starken Beeinträchtigung der Eigenschaften der Verfahrensprodukte, insbesondere deren Eigenfarbe, führt.

Bei allen Verfahren des Standes der Technik findet unter den genannten Nachteilen der Übergang von Diisocyanaten zu Biuretpolyisocyanaten statt. Dabei wird die Weiterreaktion zu Polyuretpolyisocyanaten nicht beobachtet.

Es war daher die Aufgabe der vorliegenden Erfindung, ein neues Verfahren zur Verfügung zu stellen, welches auf einfache Weise die Herstellung von hochwertigen modifizierten Polyisocyanaten gestattet, die die Vorteile der Biuretpolyisocyanate in sich vereinigen, ohne daß das Verfahren mit den genannten Nachteilen der Verfahren des Standes der Technik behaftet ist.

Diese Aufgabe konnte überraschenderweise dadurch gelöst werden, daß man bestimmte, nachstehend näher beschriebene organische Monoamine mit überschüssigen Mengen an bestimmten, nachstehend näher beschreibenen Diisocyanaten unter bestimmten nachstehend näher beschriebenen Reaktionsbedingungen umsetzt.

Gegenstand der vorliegenden Erfindung sind Verbindungen der Formel

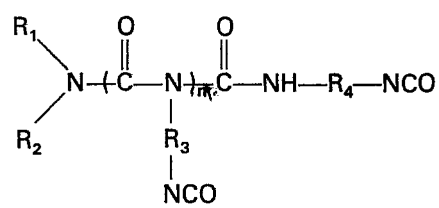

in welcher

$R_1$ und $R_2$ für gleiche oder verschiedene Reste stehen und aliphatische Kohlenwasserstoffreste mit 1—4 Kohlenstoffatomen bedeuten, wobei $R_1$ und $R_2$ zusammen mit dem Stickstoffatom auch einen Piperidinrest bilden können,

$R_3$ und $R_4$ für gleiche oder verschiedene Reste stehen und aliphatische, gegebenenfalls durch Estergruppen unterbrochene Kohlenwasserstoffreste mit 4—12 Kohlenstoffatomen oder cycloaliphatische Kohlenwasserstoffreste mit 4—15 Kohlenstoffatomen bedeuten und eine Zahl von 2—8 bedeutet.

Gegenstand der vorliegenden Erfindung ist auch ein Verfahren zur Herstellung von Verbindungen der Formel

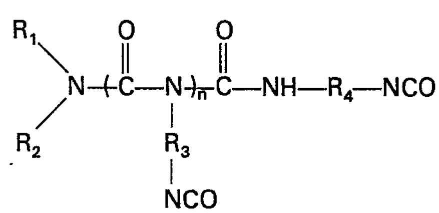

in welcher $R_1$, $R_2$, $R_3$, $R_4$ und n die genannte Bedeutung haben, dadurch gekennzeichnet, daß man

a) aliphatische sekundäre Monoamine, deren aliphatische Kohlenwasserstoff-reste 1—4 Kohlenstoffatome aufweisen oder Piperidin oder

b) Harnstoff- und/oder Biuretgruppen, sowie den bezüglich der Umsetzung indifferenten Rest der Amine aufweisende Umsetzungsprodukte der unter a) genannten Monoamine mit aliphatischen Diisocyanaten, deren aliphatischer Kohlenwasserstoffrest 4—12 Kohlenstoffatome aufweist und durch Estergruppen unterbrochen sein kann oder mit cycloaliphatischen Diisocyanaten, deren cycloaliphatischer Kohlenwasserstoffrest 4—15 Kohlenstoffatome aufweist

mit Diisocyanaten der unter b) genannten Art bei 0—140°C in Gegenwart von starken, mit Isocyanaten ein gemischtes Carbamidsäureanhydrid bildenden Säuren umsetzt, wobei man die Menge der eingesetzten Diisocyanate so wählt, daß insgesamt ein NCO/NH-Molverhältnis, bezogen auf Diisocyanate und Monoamine von 5,5:1 bis 100:1 vorliegt.

Gegenstand der vorliegenden Erfindung ist schließlich auch die Verwendung der neuen Verbindungen als Isocyanatkomponente bei der Herstellung von Polyurethankunststoffen nach dem Isocyanat-Polyadditionsverfahren.

Ausgangsmaterialien für das erfindungsgemäße Verfahren sind Monoamine der Formel

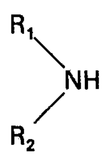

in welcher

$R_1$ und $R_2$ die bereits oben angegebene Bedeutung haben.

Beliebige Gemische der genannten Amine können ebenfalls eingesetzt werden. Konkrete Beispiele für geeignete Monoamine sind: Dimethylamin, Diäthylamin, Dipropylamin, Dibutylamin oder Piperidin.

Bei der Durchführung des erfindungsgemäßen Verfahrens entstehen aus den beispielhaft genannten Monoaminen und den nachstehend beispielhaft genannten Diisocyanaten Triuretoder höhere Polyuretgruppen aufweisende, modifizierte Polyisocyanate, in denen der bezüglich der Umsetzung indifferente Rest des Amins eingebaut ist. Diese Umsetzung verläuft über die Zwischenstufen der aus den Aminen und den Diisocyanaten sich bildenden Harnstoffgruppen aufweisenden Verbindungen bzw. sich den Harnstoffgruppen aufweisenden Verbindungen mit weiterem Diisocyanat bildenden Biuretgruppen aufweisenden Verbindungen. Zwischenstufen sind somit die sich durch Addition von einem Mol Amin und einem Mol Diisocyanat bildenden Monoharnstoff-Monoisocyanate oder auch aus zwei Mol Amin und einem Mol Diisocyanat entstehende Bisharnstoffe bzw. die sich aus diesen und weiterem Diisocyanat bildenden Biuretpolyisocyanate. Eine logische Konsequenz dieses Tatbestandes ist selbstverständlich, daß als Ausgangsmaterial gemäß einer Abänderung des erfindungsgemäßen Verfahrens auch diese beispielsweise in einem separaten Arbeitsgang aus den bei spielhaft genannten Monoaminen und den nachstehend beispielhaft genannten Isocyanaten hergestellte Zwischenprodukte eingesetzt werden können. Hierbei spielt selbstverständlich die Art der Herstellung dieser Zwischenprodukte keine Rolle. So wäre es selbstverständlich auch denkbar, als Ausgangsmaterials beim erfindungsgemäßen Verfahren das Harnstoffgruppen aufweisende Umsetzungsprodukt aus einem Mol Hexamethylen-bis-carbamidsäurechlorid und 2 Mol eines Monoamins einzusetzen, welches dem Harnstoffgruppen aufweisenden Umsetzungsprodukt aus einem Mol Hexamethylendiisocyanat und 2 Mol des gleichen Monoamins entspricht.

Für das erfindungsgemäße Verfahren geeignete Diisocyanate sind solche mit aliphatisch und/oder cycloaliphatisch gebundenen Isocyanatgruppen, insbesondere solche in denen die beiden Isocyanatgruppen über aliphatische Kohlenwasserstoffreste mit 4—12 Kohlenstoffatomen oder über cycloaliphatische Kohlenwasserstoffreste mit 4—15 Kohlenstoffatomen verbunden sind, wobei die aliphatischen oder cycloaliphatischen Kohlenwasserstoffketten durch Estergruppen unterbrochen oder substituiert sein können, wie z.B.: 1,4-Diisocyanatobutan, 1,6-Diisocyanatohexan, 2,4,4-Trimethyl-1,6-di-isocyanatohexan, 1,11-Diisocyanatoundecan, 3-Isocyanatomethyl-3,5,5-trimethylcyclohexylisocyanat, 4,4'-Cyclohexandiisocyanat, 4,4'-Dicyclohexylmethan-diisocyanat, 1,2-Bis-(isocyanatomethyl)cyclobutan oder 6-Isocyanatocapronsäure-2-isocyanatoäthylester. Bevorzugt wird Hexamethylen-diisocyanat eingesetzt.

Selbstverständlich können auch Diisocyanatgemische verwendet werden, wobei z.B. zuerst aus einem Diisocyanat mit einem monofunktionellen Amin ein Harnstoff oder ein Biuret erzeugt werden, die dann mit einem anderen Diisocyanat Polyurete bilden.

Während die Reaktion von Aminen mit Diisocyanaten zu den entsprechenden Harnstoffen bzw. Bureten zum vorbekannten Stand der Technik gehört, ist bislang noch kein technisch brauchbares Verfahren bekannt geworden, welches eine Weiterführung der zwischen Amin und Isocyanat ablaufenden Additionsreaktion über die Zwischenstufe der entsprechenden Biurete hinaus gestattet. Dies liegt an der Reaktionsträgheit der Biurete gegenüber organischen Diisocyanaten, die nur durch Verwendung geeigneter Katalysatoren überwunden werden kann. Erfindungsgemäß

wird nun auch die Weiterreaktion von Monoaminen mit überschüssigem Diisocyanat über die Harnstoff- und Biuretstufe hinaus zu Polyureten in Gegenwart von Katalysatoren durchgeführt.

Bei den erfindungsgemäß einzusetzenden Katalysatoren handelt es sich um protonenabspaltende starke Säuren, welche mit Isocyanaten, insbesondere mit aliphatischen oder cycloaliphatischen Isocyanaten, unter Bildung eines gemischten Säureanhydrids reagieren, wobei die dem Isocyanat entsprechende Carbamidsäure und die protonenabspaltende Säure die Säuren des gemischten Säureanhydrids darstellen. So reagieren derartige, für das erfindungsgemäße Verfahren geeignete Säuren HX (X = Säurerest nach Abspaltung des Protons) mit Isocyanaten Y—NCO zu Addukten der Formel Y—NH—CO—X, welche als gemischtes Anhydrid der Carbamidsäure Y—NH—COOH und der Säure HX anzusehen sind.

Beispiele geeigneter Säuren sind Halogenwasserstoffe wie z.B. Fluorwasserstoff, Chlorwasserstoff, Bromwasserstoff oder Jodwasserstoff, Chlorsulfonsäure, Fluorsulfonsäure, Schwefelsäure, Alkansulfonsäuren wie z.B. Methansulfonsäure oder perhalogenierte Alkansulfonsäuren wie z.B. Trifluormethansulfonsäure. Chlorwasserstoff ist die beim erfindungsgemäßen Verfahren bevorzugt einzusetzende Säure. Anstelle der Säuren können beim erfindungsgemäßen Verfahren selbstverständlich sowohl die den Säuren entsprechenden Ammoniumsalze mit den als Ausgangsmaterial eingesetzten Aminen oder die den Säuren entsprechenden gemischten Carbamidsäureanhydride, insbesondere Carbamidsäurechloride, der als Ausgangsmaterial eingesetzten Diisocyanate oder eines beliebigen anderen isocyanats eingesetzt werden. Im Allgemeinen werden die Katalysatoren in Mengen von 0,001—10, vorzugsweise 0,01—1,0 Gew.-%, bezogen auf das Gesamtgewicht der Reaktionspartner, eingesetzt.

Die Durchführung des erfindungsgemäßen Verfahrens insbesondere unter Verwendung der beispielhaft genannten Säuren als Katalysatoren ist bei relativ niedrigen Temperaturen im Bereich von ca. 0—140°C möglich, wobei die über die Stufe der Biuretpolyisocyanate hinausführende Reaktion zu Triuret- und Polyuretgruppen aufweisenden Umsetzungsprodukten im allgemeinen im Bereich zwischen 90 und 140°C abläuft. Die erfindungsgemäße Katalyse mit den beispielhaft genannten Säuren gestattet somit unter milden Reaktionsbedingungen die Herstellung von Polyuretgruppen aufweisenden Isocyanat-Additionsprodukten aus organischen Diisocyanaten und organischen Monoaminen bzw. aus Monoaminen und Diisocyanaten entstehenden Harnstoffgruppen oder Biuretgruppen aufweisenden Verbindungen. Bei der Durchführung des erfindungsgemäßen Verfahrens unter Verwendung von Monoaminen als Ausgangsmaterialien entstehen wegen der milden Reaktionsbedingungen keine leicht flüchtigen Nebenprodukte. Der bezüglich der erfindungsgemäßen Umsetzung indifferente Rest der Monoamine bildet somit stets einen Bestandteil der erfindungsgemäßen Verfahrensprodukte.

Bei der Durchführung des erfindungsgemäßen Verfahrens unter Verwendung von Monoaminen als Ausgangsmaterialien werden diese und die Diisocyanate im allgemeinen in Mengenverhältnissen eingesetzt, die einem NCO/NH-Molverhältnis von 5,5:1 bis 100:1, vorzugsweise 6:1 bis 30:1, entsprechen. Bei Verwendung von Harnstoffgruppen aufweisenden Ausgangsmaterialien, d.h. von aus Monoaminen und Diisocyanaten gebildeten Monoharnstoff-Monoisocyanaten bzw. Bis-Harnstoffen kommen entsprechende Mengenverhältnisse zum Einsatz, wobei zu berücksichtigen ist, daß in den genannten Harnstoffgruppen aufweisenden Ausgangsmaterialien bereits Monoamine und Diisocyanate im Molverhältnis 1:1 bzw. 2:1 vorliegen. Diese in Form der Harnstoffgruppen aufweisenden Ausgangsmaterialien vorliegenden Monoamine und Diisocyanate gehen mit in die Berechnung des Mengenverhältnisses Amin:Diisocyanat ein. Sinngemäß die gleichen Ausführungen gelten bei Verwendung von Biuretgruppen aufweisenden Ausgangsmaterialien, wie sie durch Umsetzung der genannten Harnstoffgruppen aufweisenden Zwischenprodukte mit weiterem Diisocyanat entstehen.

Zur Durchführung des erfindungsgemäßen Verfahren wird im allgemeinen wie folgt verfahren:

Man legt das Diisocyanat in einem geeigneten Reaktionsgefäß vor und gibt das Amin bei Temperaturen von 0—100°C zu. Dabei beträgt das NCO/NH-Molverhältnis im allgemeinen 6:1 bis 30:1. Feste Amine werden über einen beheitzten und flüssige Amine über einen normalen Tropftrichter dosiert. Gasförmige Amine werden eventuell zusammen mit einem Inertgasstrom in das Diisocyanat eingeleitet. Es bilden sich spontan die entsprechenden Harnstoffe. Bei Einsatz von sekundären Aminen bliebt die Reaktion bei Abwesenheit des Katalysators auf der Stufe der zumeist in überschüssigem Diisocyanat nicht vollständig löslichen Harnstoffe stehen. Die Katalysatorzugabe kann zu jedem Zeitpunkt der Reaktion erfolgen. Man kann ihn z.B. mit dem Diisocyanat vorlegen, ihn als Ammoniumsalz mit den Aminen eindosieren oder ihn erst nach Beendigung der Vorreaktion zu Harnstoff- oder Biuretisocyanaten zugeben. Danach wird das Reaktionsgemisch auf 90—140°C erhitzt und der Reaktionsverlauf durch Kontrolle der NCO-Gehaltsabnahme verfolgt. Bei Verwendung von flüchtigen Katalysatoren, z.B. Chlorwasserstoff, kann zur Vermeidung von bei höheren Temperaturen möglichen Katalysatorverlusten unter Druck gearbeitet werden.

Wenn die NCO-Abnahme dem gewünschten "Polyuretisierungsgrad" entspricht: d.h., wenn pro Aminogruppe die gewünschte Menge an NCO-Gruppen umgesetzt ist, wird die Reaktion beendet. Dies geschieht einfach durch Abkühlen des Reaktionsgemisches auf 20—50°C. Die benötigten Reaktionszeiten hängen von der Art der Ausgangsprodukte, von der Temperatur und insbesondere von der Art und Menge des verwendeten Katalysators ab. Sie betragen im allgemeinen 1—20, vorzugsweise 2—8, Stunden. Nach Beendigung der Reaktion erhält man klare, farblose bis schwach gelblich gefärbte Reaktionslösungen.

Die Reaktionen werden meistens zu einem Zeitpunkt beendet, zu dem im Mittel pro Aminogruppe ca. 3 NCO-Gruppen verbraucht sind. Die Produkte haben dann unter Berücksichtigung der Polymerhomologen eine durchschnittliche Funktionalität von 3,5. Es ist jedoch möglich, einen höheren "Polyuretisierungsgrad" zu erreichen, d.h. pro Aminogruppe 4 und mehr NCO-Gruppen umzusetzen. Allerdings nehmen die Viskositäten der Produkte dann schnell zu.

Der Katalysator wird im allgemeinen durch Andestillieren des Reaktionsgemisches im Vakuum entfernt. Bei Verwendung von Halogenwasserstoffen als Katalysatoren kann die Beseitigung, insbesondere bei kleineren Kaalysatormengen auch durch Zugabe äquimolarer Mengen Propylenoxid erfolgen. Ferner ist es möglich, den Katalysator durch z.B. Dünnschichtverdampfung zu entfernern, falls das Rohisocyanat von überschüssigem Diisocyanat befreit wird. Das Destillat der Dünnschichtdestillation, das dann neben dem Diisocyanat den Katalysator enthält, kann als Ausgangsmaterial wiederverwendet werden.

Ist die Entfernung von überschüssigem Diisocyanat vorgesehen, so erfolgt sie meistens durch Dünnschichtverdampfung; sie kann jedoch auch durch Extraktion des Reaktionsgemisches mit geeigneten Lösungsmitteln, wie z.B. Hexan, Heptan etc. erreicht werden.

Die Rohisocyanate können als olche verwendet werden. In den meisten Fällen werden sie jedoch vorzugsweise durch Dünnschichtverdampfung oder Extraktion von monomeren Isocyanatanteilen befreit. Die monomerenfreien Produkte sind hellgelbe Öle oder auch feste Harze; der NCO-Gehalt beträgt 10—22%.

Das Verfahren eignet sich vorzüglich für eine kontinuierliche Durchführung. In diesen Fällen werden mehrere Reaktionsgefäße in Form einer Kaskade hinereinandergeschaltet. Im ersten Reaktionsgefäß werden die Ausgangsprodukte Diisocyanat und Amin bei ca. 60°C vermischt. Im zweiten Reaktionsgefäß wird bei ca. 80°C der Katalysator zugegeben. Im dritten und gegebenenfalls weiteren Reaktionsgefäßen findet bei ca. 90—140°C die Weiterreaktion zum Polyisocyanat statt, wobei durch Steuerung der Temperatur und der Verweilzeit der angestrebte "Polyuretisierungsgrad" eingestellt wird.

Überschüssiges Diisocyanat und der Katalysator werden z.B. über einen Schlangenrohrverdampfer kombiniert mit nachgeschaltetem Dünnschichtverdampfer entfernt. Die aus Diisocyanat und Katalysator bestehenden Destillate werden vereinigt und wieder in den Prozeß zurückgeführt. Das Polyisocyanat wird als Rüchstand der Dünnschichtdestillation gewonnen.

Eine Extraktion des erfindungsgemäßen Verfahrensprodukts mit geeigneten Lösungsmitteln wie z.B. n-Hexan ist nicht erforderlich, da eine Abspaltung von Monoisocyanat bei der Durchführung des erfindungsgemäßen Verfahrens, welches durch Extraktion entfernt werden müßte, ausgeschlossen ist.

Bei der Durchführung des erfindungsgemäßen Verfahrens können die Eigenschaften der erhaltenen modifizierten Polyisocyanate, insbesondere deren NCO-Funktionalität, NCO-Gehalt, sowie die Viskosität nicht nur durch Wahl der geeigneten Ausgangsmaterialien sondern besonders einfach durch Einstellung des "Polyuretisierungsgrades", d.h. der Zahl der pro Aminogruppe umgesetzten NCO-Gruppen gesteuert werden.

Bei Verwendung von sekundären Monoaminen mit aliphatisch bzw. cycloaliphatisch gebundenen Aminogruppen und aliphatischen bzw. cycloaliphatischen Diisocyanaten entstehen die erfindungsgemäßen Verfahrensprodukte der eingangs angegebenen Formel. Bei der besonders bevorzugten Verwendung von Dialkylaminen mit $C_1$—$C_4$-Alkylresten bzw. von Piperidin, sowie von Hexamethylendiisocyanat als Ausgangsmaterialien bei der Durchführung des erfindungsgemäßen Verfahrens entstehen die besonders bevorzugten erfindungsgemäßen Verfahrensprodukte der genannten allgemeinen Formel, in welcher die Reste $R_1$ und $R_2$ die bereits genannte Bedeutung haben, $R_3$ und $R_4$ für Hexamethylenreste stehen und n für eine Zahl von 2 bis 8 steht.

Die erfindungsgemäßen Verfahrensprodukte können insbesondere als Isocyanatkomponente bei der Herstellung von Polyurethankunststoffen nach dem Isocyanat-Polyadditionsverfahren eingesetzt werden. Sie eignen sich sowohol zur Herstellung von Polyurethan-Schaumstoffen, als auch zur Herstellung von Elastomeren, Beschichtungen oder Verklebungen. Insbesondere bei Verwendung der erfindungsgemäßen Verfahrensprodukte für das erstgenannte Einsatzgebeit erübrigt sich oft ein Abdestillieren des überschüssigen Diisocyanats nach Beendigung der erfindungsgemäßen Umsetzung. Die monomerenfreien erfindungsgemäßen Verfahrensprodukte stellen hervorragende Rohstoffe zur Herstellung hochwertiger, wetterfester und lichtechter Lackierungen dar.

Bezüglich der Verwendung der erfindungsgemäßen Verfahrensprodukte als "Lackisocyanate" ist insbesondere ihre ausgezeichnete Verträglichkeit mit handelsüblichen Polyhydroxy-Polyacrylaten hervorzuheben. Ein

weiterer Vorteil der erfindungsgemäßen Verfahrensprodukte gegenüber bekannten Biuretpolyisocyanaten ist darin zu sehen, daß sie gegen Monomerenrückspaltung stabil sind, d.h. daß auch während der Lagerung bei erhöhter Temperatur (50°C) der Monomerengehalt der erfindungsgemäßen Polyisocyanate nicht ansteigt.

BEISPIEL 1

In einem 4 l-Vierhalskolben mit Rührer, Rückflußkühler und Kontaktthermometer wurden in 3024 g (18 Mol) 1,6-Diisocyanatohexan unter Stickstoffüberlagerung 135 g (3 Mol) Dimethylamin eingeleitet, wobei die Temperatur im Reaktionsgefäß auf ca. 60°C anstieg. Es bildete sich der entsprechende Harnstoff, der bis auf geringe Restmengen nach Beendigung der Aminzugabe gelöst war. Nun wurden 7 g (0,19 Mol) Chlorwasserstoff in 100 g 1,6-Diisocyanatohexan dem Reaktionsgemisch zugefügt und die Temperatur auf 100°C erhöht. Der NCO-Gehalt der nun klaren Reaktionslösung betrug 44% (entsprechend einem Verbrauch von 1 NCO-Gruppe pro Aminogruppe). Nach 2 Stunden war der NCO-Gehalt auf 40% (entsprechend einem Verbrauch von 2 NCO-Gruppen pro Aminogruppe) und nach 4 Stunden auf 35% (entsprechend einem Verbrauch von 3,3 NCO-Gruppen pro Aminogruppe, entsprechend n = 2,3) gesunken. Die Reaktionslösung wurde auf 50°C abgekühlt und zur Bindung des hydrolysierbaren Chlors mitd 11 g (0,19 Mol) Propylenoxid versetzt. Die nachfolgende Dünnschichtdestillation ergab 1600 g 1,6-Diisocyanatohexan als Destillat und 1540 g Polyuretpolyisocyanat als Rückstand (NCO-Gehalt = 19,5%; Viskosität bei 25°C = 5000 mPa.s; Restgehalt an monomerem 1,6-Diisocyanatohexan = 0,60%).

Anhand der Kontrolle des Monomerengehalts von Produktproben, die über längere Zeit bei 50°C gelagert wurden, zeigte sich, daß das Polyisocyanat gegen Monomerenrückspaltung stabil ist.

| Lagerung (in Wochen) | 1,6-Diisocyanatohexan (%) |
|---|---|
| — | 0,60 |
| 1 | 0,72 |
| 4 | 0,68 |
| 6 | 0,67 |

BEISPIEL 2

Analog Beispiel 1 wurden 3024 g (18 Mol) 1,6-Diisocyanatohexan unter Zusatz von 7 g (0,19 Mol) Chlorwasserstoff mit 225 g (5 Mol) Dimethylamin umgesetzt. Nach 7 Stunden betrug der NCO-Gehalt des Reaktionsgemisches 27,4% (entsprechend einem Verbrauch von 3 NCO-Gruppen pro Aminogruppe (n = 2)). Nach der Dünnschichtverdampfung erhielt man neben 1000 g 1,6-Diisocyanatohexan 2150 g Polyisocyanat (NCO-Gehalt = 16,2%; Viskosität bei 25°C = 30 000 mPa.s; Restmonomerengehalt = 0,3%).

BEISPIEL 3

Analog Beispiel 1 wurden 1008 g (6 Mol) 1,6-Diisocyanatohexan unter Zusatz von 3 g (0,08 Mol) Chlorwasserstoff mit 45 g (1 Mol) Dimethylamin umgesetzt. Nach 6 Stunden war der NCO-Gehalt des Reaktionsgemisches auf 28% gesunken (entsprechend einem Umsetzungsgrad von 5 NCO-Gruppen pro Aminogruppe (n = 4)). Die Viskosität des Rohisocyanats betrug 200 mPa.s/25°C.

Beispiel 4

Analog Beispiel 1 wurden die in der Tabelle zusamengestellten Umsetzungen durchgeführt:

| Isocyanat g (Mol) | Amin g (Mol) | Isocyanat / Amin-Molverhältnis | Katalysator | Ausbeute (g) Polyisocyanat | NCO (%) | $\eta$ 25°C (mP. s) | Monomerengehalt (%) | n |
|---|---|---|---|---|---|---|---|---|
| 3024 (18) HDI * | 387 (3) Dibutylamin | 6:1 | 4 g HCl | 1670 | 17 | 1400 | 0,68 | 2,3 |
| 1008 (6) HDI | 85 (1) Piperidin | 6:1 | 3 g HCl | 430 | 17,4 | 6500 | 0,45 | 2,1 |
| 3024 (18) HDI | 135 (3) Dimethylamin | 6:1 | 19 g Methansulfonsäure | 1430 | 17,4 | 11000 | 0,31 | 2,5 |
| 1008 (6) HDI | 45 (1) Dimethylamin | 6:1 | 7 g Dimethylaminhydrochlorid | 500 | 18,4 | 7000 | 0,53 | 2,2 |
| 2260 (10) X** | 75 (1,67) Dimethylamin | 6:1 | 4 g HCl | 1010 | 14,3 | 4000 | 0,70 | 2,0 |
| 1776 (8) IPDI**** 672 (4) HDI | 90 (2) Dimethylamin | 6:1 | 3 g HCl | 1200 | 17 | 1600**** | 0,62 | 2,1 |

Erklärungen zur Tabelle Beispiel 4

* HDI entspricht 1,6-Diisocyanatohexan

** X entspricht 6-Isocyanatocapronsaure-2-isocyanatoäthylester

*** IPDI entspricht 3-Isocyanatomethyl-3,5,5-trimethylcyclohexylisocyanat

**** in Äthylglycolacetat/Xylol (1:1)

BEISPIEL 5

(Verwendungsbeispiel)

154 g einer 65%-igen Lösung eines stark verzweigten Polyesters auf Basis Phthalsäureanhydrid und Trimethylolpropan (Hydroxylgehalt 8%) in Äthylglycolacetat/Xylol (1:1) wurden nach Zugabe von 1 g eines tertiären Amins als Katalysator und 0,4 g Cellulosebutyrat-propionat als Verlaufmittel mit 220 g eines Lösungsmittelgemisches aus Methyläthylketon, Butylacetat, Äthylglycolacetat und Toluol (4:1:4:1) verdünnt. Hierzu wurden 135 g einer 75 %igen Lösung des Polyisocyanats aus Beispiel 1 in Äthylglycolacetat/Xylol (1:1) gegeben (NCO/OH-Molverhältnis = 1:1). Die fertige Lacklösung wurde dann auf Stahlbleche aufgetragen, wo die Lackfilme bei Raumtemperatur aushärteten. Die durchgehärteten Klarlackfilme waren kratzfest, elastisch und gegen Lösungsmittel wie Toluol, Äthylglycolacetat, Äthylacetat oder Aceton beständig. Sie hatten ferner folgende Eigenschaften:

| | |
|---|---|
| Schichtdicke | ca. 50 $\mu$ |
| Erichsentiefung (DIN 53156) | |
| nach 2 Tagen | 8,8 mm |
| nach 5 Tagen | 8,5 mm |
| nach 14 Tagen | 8,4 mm |
| Pendelhärte (DIN 53157) | |
| nach 2 Tagen | 215 Sekunden |
| nach 5 Tagen | 225 Sekunden |
| nach 14 Tagen | 223 Sekunden |

BEISPIEL 6

(Verwendungsbeispiel)

Es wurden 154 g der im Beispiel 5 beschriebenen Polyesterlösung mit 100 g Titandioxid (Rutiltyp) zu einer Paste verarbeitet. Dieser Paste wurden neben Katalysator und Verlaufmittel 120 g des schon beschriebenen Lösungsmittelgemisches zugefügt. Die so erhaltene Mischung wurde mit 135 g einer 75 %igen Lösung des Polyisocyanats aus Beispiel 1 in Äthylglycolacetat/Xylol (1:1) versetzt und in dünner Schicht auf Stahlbleche aufgetragen. Die pigmenthaltigen Lackfilme härteten bei Raumtemperatur durch. Sie zeichneten sich durch Kratzfestigkeit und Lösungsmittelresistenz aus und hatten verglichen mit den Klarlackfilmen folgende Eigenschaften:

| | |
|---|---|
| Schichtdicke | ca. 50 $\mu$ |
| Erichsentiefung (DIN 53156) | |
| nach 2 Tagen | 9,1 mm |
| nach 6 Tagen | 8,5 mm |
| nach 14 Tagen | 8,9 mm |
| Pendelhärte (DIN 53157) | |
| nach 2 Tagen | 175 Sekunden |
| nach 5 Tagen | 180 Sekunden |
| nach 14 Tagen | 179 Sekunden |

**Patentansprüche**

1. Verbindungen der Formel

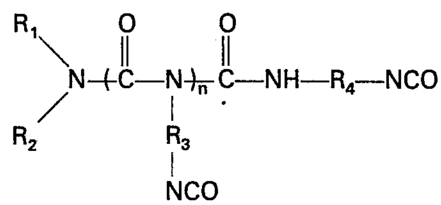

in welcher

$R_1$ und $R_2$ für gleiche oder verschiedene Reste stehen und aliphatische Kohlenwasserstoffreste mit 1—4 Kohlenstoffatomen bedeuten, wobei $R_1$ und $R_2$ zusammen mit dem Stickstoffatom auch einen Piperidinrest bilden können,

$R_3$ und $R_4$ für gleiche oder verschiedene Reste stehen und aliphatische, gegebenenfalls durch Estergruppen unterbrochene Kohlenwasserstoffreste mit 4—12 Kohlenstoffatomen oder cycloaliphatische Kohlenwasserstoffreste mit 4—15 Kohlenstoffatomen bedeuten und

n eine Zahl von 2—8 bedeutet.

2. Verbindungen der Formel gemäß Anspruch 1, wobei $R_1$, $R_2$ und n die in Anspruch 1 genannte Bedeutung haben und $R_3$ und $R_4$ jeweils für Hexamethylenreste stehen.

3. Verfahren zur Herstellung von Verbindungen der Formel

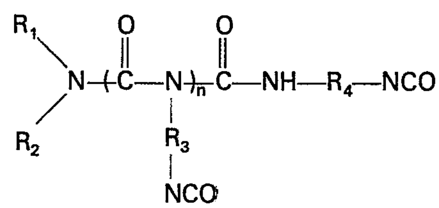

in welcher $R_1$, $R_2$, $R_3$, $R_4$ und n die in Anspruch 1 genannte Bedeutung haben, dadurch gekennzeichnet, daß man

a) aliphatische sekundäre Monoamine, deren aliphatische Kohlenwasserstoffreste 1—4 Kohlenstoffatome aufweisen oder Piperidin oder

b) Harnstoff- und/oder Biuretgruppen, sowie den bezüglich der Umsetzung indifferenten Rest der Amine aufweisende Umsetzungsprodukte der unter a) genannten Monoamine mit aliphatischen Diisocyanaten, deren aliphatischer Kohlenwasserstoffrest 4—12 Kohlenstoffatome aufweist und durch Estergruppen unterbrochen sein kann oder mit cycloaliphatischen Diisocyanaten, deren cycloaliphatischer Kohlenwasserstoffrest 4—15 Kohlenstoffatome aufweist

mit Diisocyanaten der unter b) genannten Art bei 0—140°C in Gegenwart von starken, mit Isocyanaten ein gemischtes Carbamidsäureanhydrid bildenden Säuren umsetzt, wobei man die Menge der eingesetzten Diisocyanate so wählt, daß insgesamt ein NCO/NH-Molverhältnis, bezogen auf Diisocyanate und Monoamine von 5,5 : 1 bis 100 : 1 vorliegt.

4. Verwendung der Verbindungen gemäß Anspruch 1 als Isocyanatkomponente bei der Herstellung von Polyurethankunststoffen nach dem Isocyanat-Polyadditionsverfahren.

**Claims**

1. Compounds of the formula

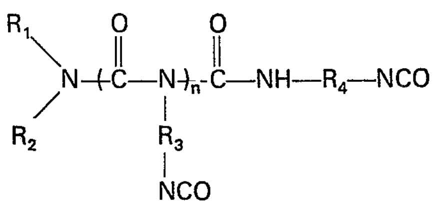

in which

$R_1$ and $R_2$ represent identical or different radicals and denote aliphatic hydrocarbon radicals with 1—4 carbon atoms, $R_1$ and $R_2$, together with the nitrogen atom, also being able to form a piperidine radical,

$R_3$ and $R_4$ represent identical or different radicals and denote aliphatic hydrocarbon radicals with 4—12 carbon atoms, optionally interrupted by ester groups, or they denote cycloaliphatic hydrocarbon radicals with 4—15 carbon atoms and

n denotes an integer from 2 to 8.

2. Compounds of the formula according to Claim 1, $R_1$, $R_2$ and n having the meaning mentioned in Claim 1 and $R_3$ and $R_4$ each representing hexamethylene radicals.

3. A process for the preparation of compounds of the formula

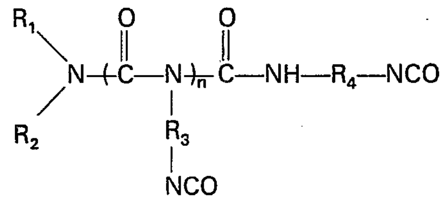

in which $R_1$, $R_2$, $R_3$, $R_4$ and n have the meaning mentioned in Claim 1, characterised in that

a) aliphatic secondary monoamines, the aliphatic hydrocarbon radicals of which have 1—4 carbon atoms, or piperidine or

b) reaction products containing urea and/or biuret groups and the amine radical inert to the reaction, from the reaction of the monoamines named under a) with aliphatic diisocyanates, the aliphatic hydrocarbon radical of which contains 4—12 carbon atoms and can be interrupted by ester groups, or with cycloaliphatic diisocyanates, the cycloaliphatic hydrocarbon radical of which contains 4—15 carbon atoms,

are reacted with diisocyanates of the kind mentioned under b), at 0—140°C in the presence of strong acids which form a mixed carbamic acid anhydride with isocyanates; the quantity of diisocyanates used is chosen so that there is altogether an NCO/NH molar ratio of 5.5 : 1 to 100 : 1, related to the diisocyanates and and monoamines.

4. The use of the compounds according to Claim 1 as isocyanate components in the production of polyurethane plastics according to the isocyanate polyaddition process.

**Revendications**

1. Composés répondant à la formule générale:

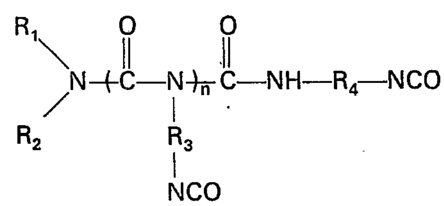

dans laquelle

$R_1$ et $R_2$ sont identiques ou différents et représentent chacun un reste d'hydrocarbure aliphatique en $C_1$—$C_4$, $R_1$ et $R_2$, pris ensemble avec l'atome d'azote, pouvant aussi former un reste pipéridine;

$R_3$ et $R_4$ sont identiques ou différents et représentent chacun un reste d'hydrocarbure aliphatique en $C_4$—$C_{12}$ éventuellement interrompu par des groupes ester, ou un reste d'hydrocarbure cycloaliphatique en $C_4$—$C_{15}$; et

n est un nombre entier compris entre 2 et 8 inclus.

2. Composés selon la revendication 1, caractérisé en ce que $R_1$, $R_2$ et n sont tels que définis ci-dessus et $R_3$ et $R_4$ représentent chacun un reste hexaméthylène.

3. Procédé pour la préparation de composés de formule

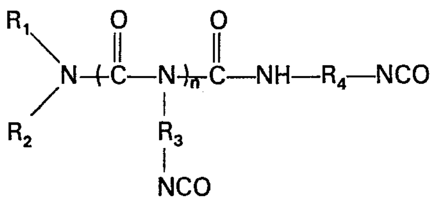

dans laquelle $R_1$, $R_2$, $R_3$, $R_4$ et n sont tels que définis à la revendication 1, caractérisé en ce que l'on fait réagir

a) des monoamines secondaires aliphatiques à restes hydrocarbure aliphatiques en $C_1$—$C_4$ ou la pipéridine, ou

b) des produits de réaction des monoamines mentionnées sous a) avec des diisocyanates aliphatiques à reste hydrocarbure aliphatique en $C_4$—$C_{12}$ et pouvant éventuellement être interrompu par des groupes ester, ou avec des diisocyanates cycloaliphatiques à reste hydrocarbure cycloaliphatique en $C_4$—$C_{15}$, ces produits de réaction présentant des groupes urés et/ou biurat ainsi que le reste des amines indifférent vis-à-vis de la réaction,

avec des diisocyanates du type mentionné sous b) à 0—140°C en présence d'acides forts formant avec les isocyanates un anhydride carbamique mixte, en choisissant la quantité de diisocyanate utilisé de telle sorte que le rapport molaire global NCO/NH, rapporté aux diisocyanates et aux monoamines, soit de 5,5 : 1 à 100 : 1.

4. Utilisation des composés la revendication 1 comme composant isocyanate dans la préparation de matière plastique de polyuréthannes par le procédé de polyaddition des isocyanates.